# FASCICULE DE BREVET EUROPEEN

(11) **EP 2 500 998 B1**
(45) Date de publication et mention de la délivrance du brevet: **11.06.2014**
(21) Numéro de dépôt: 12153640.3
(22) Date de dépôt: 02.02.2012
(51) Int. Cl.: H01R 107/00, H01R 24/58

(54) **Fiche de connexion électrique pour sonde multipôle de dispositif médical implantable actif**
Elektrischer Anschlussstift für multipolare Sonde einer aktiven implantierbaren medizinischen Vorrichtung
Electrical connection plug for multipole probe of an active implantable medical device

(30) Priorité: 16.03.2011 FR 1152166
(43) Date de publication de la demande: 19.09.2012
(73) Titulaire: Sorin CRM SAS, 92140 Clamart Cedex (FR)
(72) Inventeur: Régnier, Willy, 91160 Longjumeau (FR); Viatge, Hélène, 92120 Montrouge (FR)
(74) Mandataire: Dupuis-Latour, Dominique

(56) Documents cités:
- EP-A1- 1 641 084
- EP-A2- 2 090 332
- US-A1- 2005 221 671

## Description

L'invention concerne de façon générale les "dispositifs médicaux implantables actifs" tels que définis par la directive 90/385/CEE du 20 juin 1990 du Conseil des communautés européennes.

Cette définition inclut en particulier les implants cardiaques chargés de surveiller l'activité cardiaque et de générer des impulsions de stimulation, de défibrillation et/ou de resynchronisation en cas de trouble du rythme détecté par l'appareil. Elle inclut aussi les appareils neurologiques, les implants cochléaires, les pompes de diffusion de substances médicales, les capteurs biologiques implantés, etc.

Ces dispositifs comportent un boîtier généralement désigné "générateur", raccordé électriquement et mécaniquement à une ou plusieurs "sondes" munie(s) d'électrodes destinées à venir en contact avec les tissus sur lesquels on souhaite appliquer des impulsions de stimulation et/ou recueillir un signal électrique : myocarde, nerf, muscle, ...

Il existe des systèmes de connexion normalisés permettant de garantir l'interchangeabilité des sondes et des générateurs produits par différents fabricants.

Ainsi, les standard dits "IS-1" et "IS-4" définissent un certain nombre de caractéristiques dimensionnelles et électriques relatives aux sondes de délivrance d'impulsions de stimulation. Pour les sondes de défibrillation ou de cardioversion, où les contraintes électriques sont plus sévères compte tenu de l'énergie élevée devant transiter du générateur à la sonde, les standards dit "DF-1" et "DF-4" définissent les caractéristiques dimensionnelles et électriques du système de connexion.

L'invention concerne plus particulièrement une fiche de connexion électrique multipôle pour une sonde telle que par exemple une sonde mono- corps pourvue à la fois d'électrodes de stimulation et de choc.

La complexité de ces sondes, qui intègrent déjà les contraintes spécifiques en termes d'énergie électrique liées à l'une ou l'autre des utilisations, stimulation ou choc, est encore accrue par le développement des dispositifs multisite et des capteurs intracardiaques, tels que les capteurs de pic d'accélération endocavitaire (PEA). Ceci conduit, du point de vue de la connectique, à une multiplication des fiches de connexion, avec au surplus des standards différents selon les fiches.

On comprend l'avantage que peut représenter une fiche unique, soumise à un standard unique, portant une pluralité de contacts électriques permettant d'assurer simultanément l'établissement des connexions aux diverses bornes du générateur pour tous les niveaux d'énergie, qu'il s'agisse du recueil de signaux de dépolarisation, de l'application d'impulsions de stimulation ou de l'envoi d'une énergie de choc ou de défibrillation.

Dans ce cadre, il a été proposé une fiche de connexion unique du type "isodiamètre", c'est-à-dire de forme cylindrique uniforme, destinée à être insérée dans une cavité homologue de générateur.

Le EP 1 641 084 A1 (ELA Medical) décrit une telle fiche de connexion isodiamètre dont la surface cylindrique extérieure présente un empilement de zones de contact électrique annulaires, réalisées par des bagues cylindriques conductrices, et de zones cylindriques isolantes destinées à isoler électriquement les bagues conductrices.

Par ailleurs, chaque contact électrique dans la cavité du générateur doit être isolé des autres et de l'environnement hors cavité par des joints. Initialement prévus sur la sonde, ces joints sont maintenant placés dans la cavité du fait que, pour les sondes de défibrillation notamment, des tensions assez importantes parcourent les éléments de contact. Il est donc primordial que les fiches de connexion de sondes multipôle soient dimensionnellement stables dans le temps et respectent avec des tolérances précises la description géométrique des normes imposées. Ces exigences permettent d'assurer que les zones de contact et les zones isolantes coïncident avec les zones correspondantes des cavités des générateurs aussi bien lors de l'insertion des fiches de connexion dans les cavités qu'au cours de la vie du dispositif médical considéré.

Dans cette perspective, deux paramètres essentiels sont à prendre en compte, à savoir, d'une part, l'état de surface des zones de contact électrique et des zones isolantes, et, d'autre part, la coaxialité de ces zones le long de la fiche de connexion. Ces paramètres sont en effet déterminants quant à la qualité du contact électrique dans la cavité du générateur et de l'étanchéité du système.

A ces contraintes s'ajoute la difficulté de réalisation d'une fiche de connexion avec un diamètre constant sur toute la longueur de la pièce et de multiples contacts électriques, ce qui soulève beaucoup de problèmes de fabrication. De plus, la contrainte d'un diamètre extérieur faible (3,2 mm selon la norme ISO 27 186) limite les possibilités de conception, de sorte que l'impact du respect de tolérances aussi serrées dans un cadre de production industrielle peut être considérable en termes de temps et de coût.

A cet égard, la fiche de connexion décrite dans le EP 1 641 084 A1 précité n'est pas totalement satisfaisante car elle ne garantit pas une coaxialité parfaite des différentes zones. En effet, dans cette fiche connue, les zones de contact et les zones isolantes sont définies par des pièces élémentaires cylindriques dont l'alignement axial et angulaire est obtenu par des piges longitudinales emmanchées dans des alésages de section homologue pratiqués dans chacune des pièces élémentaires. Cependant, le jeu fonctionnel minimum existant entre les piges et les alésages pour permettre l'empilement des pièces élémentaires induit un défaut de coaxialité de l'ensemble, nuisible au contact électrique et à l'étanchéité de la fiche de connexion à l'intérieur de la cavité du générateur.

Le US 2005/221671 A1 propose, pour résoudre cette difficulté, une fiche de connexion électrique pour sonde multipôle de dispositif médical implantable actif, ladite fiche présentant une surface extérieure cylindrique comprenant une pluralité de zones de contact électrique annulaires réparties axialement formées de bagues cylindriques conductrices, les zones de contact électrique étant séparées alternativement par une pluralité de zones cylindriques isolantes intercalaires. La fiche de connexion comprend en outre un noyau central monobloc isolant de forme générale cylindrique présentant une pluralité de surfaces latérales cylindriques de centrage coaxiales, une bague conductrice étant placée sur au moins une surface latérale de centrage.

La coaxialité recherchée des bagues conductrices résulte de celle des surfaces de centrage, obtenues lors de la fabrication du noyau central monobloc. Le caractère monobloc, donc sans jeu fonctionnel, du noyau central garantit la stabilité à long terme de la coaxialité des bagues. Toutefois, la structure décrite impose le soudage du fil de liaison "en aveugle" dans un trou traversant de la bague conductrice, donc sans possibilité de contrôle visuel de l'intégrité de la soudure sur toute l'étendue de celle-ci. Il subsiste également le problème du positionnement correct des différentes bagues (à savoir les bagues conductrices munies de leur fil soudé et les bagues isolantes) au moment de l'assemblage de la fiche, en respectant les contraintes d'alignement longitudinal et de centrage des bagues (pour une coaxialité parfaite des zones de contact et des zones isolantes), avec une reproductibilité et une fiabilité suffisantes, sans augmentation sensible des coûts de production et au moyen de pièces et de procédés simples à fabriquer et à maîtriser, ceci dans la recherche d'une solution industrielle avec une rentabilité et une efficacité permettant la production en grandes quantités.

A cet effet, selon l'invention, le noyau central comprend une pluralité de logements recevant une pluralité correspondante de cosses conductrices intermédiaires sur lesquelles sont soudés des fils de liaison, les cosses étant soudées à des bagues conductrices respectives. Avantageusement, les cosses intermédiaires présentent une rainure d'insertion du fil de liaison, alignée dans l'axe du noyau ou transversalement à cet axe.

De façon pratique, les bagues conductrices sont amenées en position sur les zones de contact électrique respectives en les enfilant le long du noyau central. Afin de faciliter cette opération, il est prévu par l'invention que les surfaces latérales de centrage comportent un épaulement de positionnement longitudinal des bagues conductrices. Les épaulements des surfaces latérales jouent en quelque sorte le rôle de butée pour les bagues conductrices. On obtient ainsi une très bonne précision dans la mise en place des bagues.

Afin de faciliter l'introduction des bagues sur le noyau, il y a avantage, selon l'invention, à ce que le noyau central présente un méplat longitudinal pour une introduction des bagues conductrices sur les surfaces latérales de centrage par déformation élastique réversible. Il suffit donc de donner aux bagues, par simple pression, une forme légèrement ovale pour les introduire sur le noyau, en passant éventuellement au-dessus des épaulements de positionnement, et de les faire glisser axialement jusqu'à leur emplacement, puis de relâcher la pression exercée pour qu'elles retrouvent leur forme annulaire initiale et viennent s'appuyer sur les surfaces latérales de centrage.

Par ailleurs, les zones isolantes intercalaires sont avantageusement constituées par des bagues isolantes. Dans ce cas, les bagues isolantes sont enfilées sur le noyau central alternativement avec les bagues conductrices. On peut également envisager le surmoulage d'une bague isolante sur une bague conductrice.

En variante, les zones isolantes intercalaires sont réalisées par surmoulage ou injection d'un matériau isolant. Ce procédé consiste à remplir les espaces créés entre les bagues conductrices pour réaliser une pièce isodiamètre qui respecte en particulier la norme ISO 27186.

Selon un mode de réalisation avantageux, le noyau central est réalisé par moulage d'un matériau isolant, comme la polyétheréthercétone (PEEK) ou le *Tecothane* (marque déposée).

En effet, de par sa forme globalement cylindrique, le noyau central permet un démoulage dit "naturel", suivant l'axe d'ouverture du moule, sans tiroirs ni cales. Ce procédé par moulage permet d'obtenir des caractéristiques dimensionnelles très précises concernant notamment les surfaces de centrage et leur coaxialité. Bien évidemment, un même moule peut être utilisé pour réaliser un grand nombre de pièces. Il en résulte une excellente reproductibilité des dimensions d'une pièce à l'autre.

Cependant, l'avantage du procédé par moulage n'exclut pas pour autant que le noyau central puisse être également fabriqué par usinage ou par toute autre manière de produire des pièces isolantes.

Un autre problème que l'invention doit prendre en compte se rapporte au maintien en position des fils de liaison qui s'étendent axialement le long du noyau central, en provenant de la sonde pour aboutir au niveau des contacts électriques de la fiche de connexion avec le générateur. En effet, des fils flottants peuvent se toucher ou toucher une bague conductrice. Dans ce but, l'invention prévoit que des moyens de maintien de fils de liaison conducteurs le long du noyau central cylindrique sont ménagés sur les surfaces latérales de centrage. En particulier, les moyens de maintien comprennent une encoche longitudinale ménagée sur les surfaces latérales de centrage et destinée à appliquer un fil de liaison contre le noyau cylindrique. Avantageusement, les moyens de maintien comprennent en outre au moins une deuxième surface latérale de centrage destinée à retenir un fil de liaison dans au moins une encoche longitudinale.

Ainsi, les surfaces latérales de centrage ont une double fonction, celle d'assurer le centrage proprement dit des bagues conductrices et celle de maintenir en place les fils de liaison.

On va maintenant décrire un exemple de réalisation de l'invention, en référence aux dessins annexés où les mêmes références numériques désignent d'une figure à l'autre des éléments identiques ou fonctionnellement semblables.
La Figure 1 est une vue en perspective d'une sonde multipôle munie d'une fiche de connexion selon l'invention.
La Figure 2 est une coupe longitudinale de la sonde de la Figure 1.
La Figure 3 est une vue en perspective du noyau central de la fiche de connexion montrée aux Figures 1 et 2.
La Figure 4 est une vue de détail du noyau central de la Figure 3.
La Figure 5 est une vue de face du noyau central de la Figure 3 disposé sur un support.
La Figure 6 est une vue en perspective d'une variante de réalisation du noyau central de la Figure 3.
La Figure 7 est une vue de face du noyau central de la Figure 3 muni d'un méplat longitudinal.
Les Figures 8a et 8b sont des vues en perspective d'un premier mode de montage d'un fil de liaison sur une cosse intermédiaire.
Les Figures 9a et 9b sont des vues en perspective d'un second mode de montage d'un fil de liaison sur une cosse intermédiaire.
La Figure 10 est une vue de dessus du noyau central de la Figure 3 équipé de cosses intermédiaires
La Figure 11 est une vue en perspective du noyau central de la Figure 10 représentant des moyens de maintien des fils de liaison.
La Figure 12 est une vue en coupe selon la ligne A-A de la Figure 10.
La Figure 13 est une vue en perspective d'une fiche de connexion selon l'invention en cours de montage des bagues conductrices.
La Figure 14 est une vue en coupe selon la Figure 12 de la fiche de connexion de la Figure 13.
La Figure 15 est une vue en perspective d'une fiche de connexion selon l'invention après montage des bagues conductrices.
La Figure 16 est une vue en perspective de la fiche de connexion après surmoulage des zones isolantes intercalaires.
La Figure 17 est une vue en coupe de la fiche de la Figure 16 à travers une zone de contact.
La Figure 18 est une vue en coupe de la fiche de la Figure 16 à travers une zone isolante intercalaire.
La Figure 19 est une vue en perspective illustrant une variante de réalisation des zones isolantes intercalaires.
La Figure 20 est une vue en perspective illustrant une autre variante de réalisation des zones isolantes intercalaires.
La Figure 21 est une vue en coupe du noyau central de la Figure 7 montrant un mode d'introduction des bagues conductrices par déformation élastique.

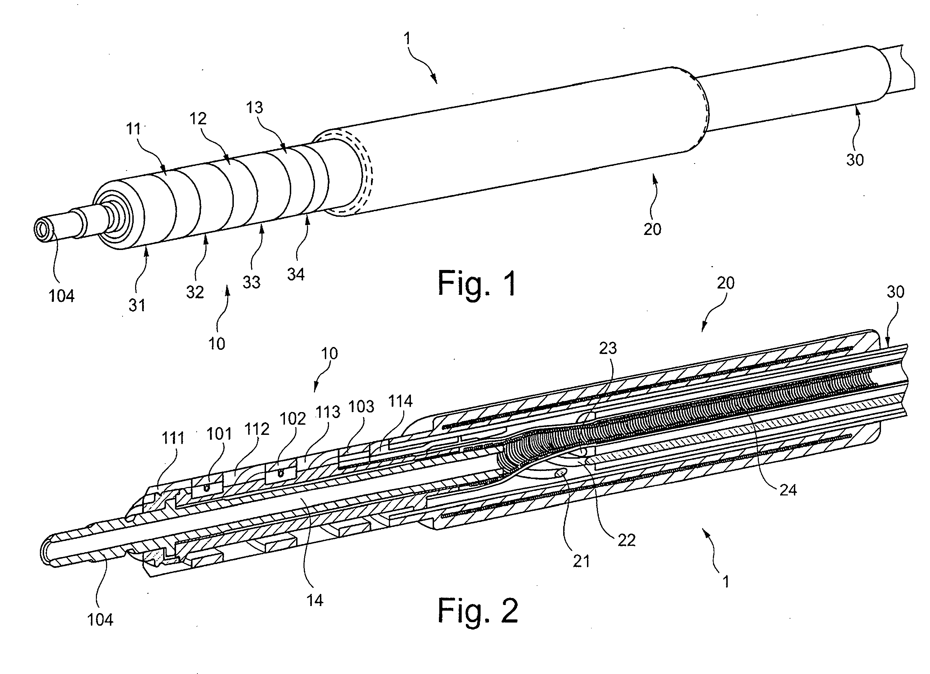

Sur les Figures 1 et 2, on a représenté l'extrémité proximale d'une sonde multipôle 1 d'un dispositif médical du type implantable, par exemple un stimulateur cardiaque, défibrillateur ou resynchroniseur.

La sonde 1 comporte un corps de sonde 20 dans lequel sont disposés divers conducteurs de liaison s'étendant, par l'intermédiaire d'une fiche 10 de connexion, entre un générateur d'impulsions électriques (non représenté) et les pôles de la sonde 1 placés à l'extrémité distale du corps 20. Dans l'exemple de réalisation proposé sur les Figures 1 et 2, quatre conducteurs de liaison sont représentés, dont un conducteur creux 24, par exemple un câble enroulé en spirale, relié électriquement à une broche axiale 104 assurant un contact électrique axial avec le générateur, et présentant en son centre une lumière communiquant avec une lumière correspondante 14 pratiquée dans la broche axiale 104. Cette disposition permet l'introduction d'un mandrin pour le guidage de la sonde par le praticien au moment de son implantation chez le patient ou pour le passage d'un système de fixation de la sonde par vis.

Le conducteur creux 24 est logé à l'intérieur d'une gaine souple 30 en matériau isolant tel qu'une silicone dont les propriétés de résistance à la fatigue sont excellentes. En revanche, pour faciliter son introduction dans le réseau veineux, la gaine 30 est pourvue extérieurement d'un revêtement en matériau à faible coefficient de frottement, par exemple du polyuréthanne.

Outre le conducteur creux 24, la gaine 30 contient, dans l'exemple non limitatif de la Figure 2, trois autres conducteurs de liaison que sont les fils 21, 22, 23 dont les extrémités proximales sont raccordées respectivement à trois zones 11, 12, 13 de contact électrique annulaires réparties axialement le long de la fiche 10 de connexion. Comme on peut l'observer sur les Figures 1 et 2, cette dernière est du type multipolaire cylindrique, enfichable d'un seul geste dans une cavité homologue du générateur. Ce mode d'enfichage assure simultanément le raccordement électrique des diverses électrodes situées aux pôles de la sonde 1 aux bornes correspondantes du générateur. Une telle fiche de connexion multipolaire est notamment décrite dans le EP 1 641 084 A1 précité.

La fiche 10 des Figures 1 et 2 forme un ensemble "isodiamètre", c'est-à-dire dans lequel les zones 11, 12, 13 de contact électriques annulaires et des zones 31, 32, 33, 34 isolantes intercalaires séparant alternativement les zones de contact présentent une surface extérieure cylindrique lisse. Dans le mode de réalisation des Figures 1 et 2, les zones 11, 12, 13, sont réalisées par des bagues cylindriques conductrices 101, 102, 103 auxquelles sont raccordés les fils 21, 22, 23 de liaison d'une manière qui sera expliquée plus loin.

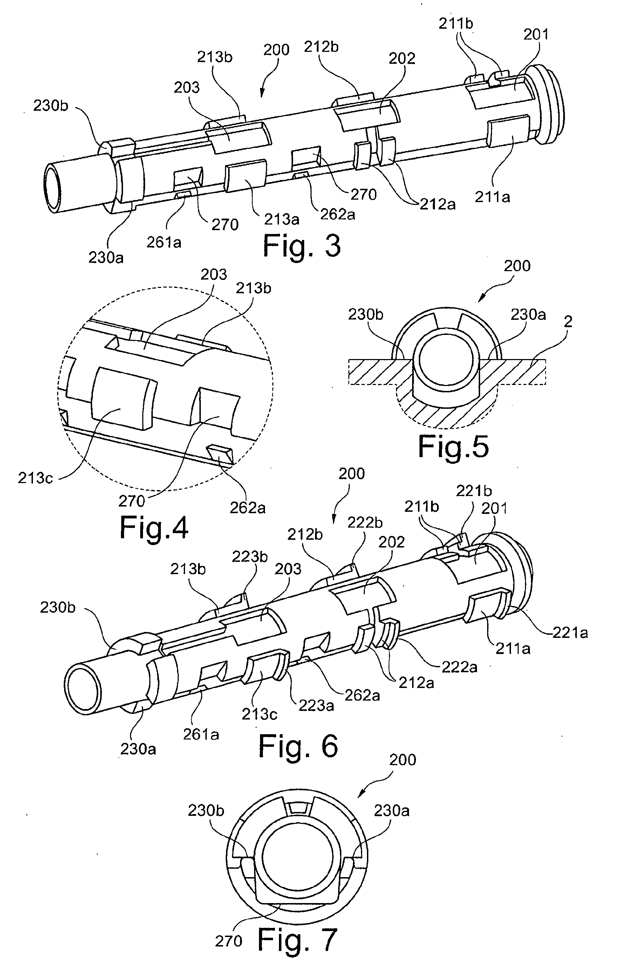

La fiche 10 de connexion est organisée autour d'un noyau central monobloc 200 creux de forme générale cylindrique dont un exemple de réalisation est montré sur la Figure 3. Comme cela a été indiqué plus haut, le noyau 200 peut être avantageusement réalisé par moulage naturel d'un matériau isolant tel que PEEK (polyétheréthercétone) ou Tecothane (marque déposée).

Des évidements notés génériquement 270 sur la Figure 3 sont prévus sur le corps du noyau 200 afin d'en simplifier le démoulage.

Le noyau central 200 est principalement caractérisé par la présence de surfaces latérales 211a, 211b, 212a, 212b, 213a, 213b de centrage coaxiales, permettant d'obtenir de manière très simple et à faible coût une coaxialité parfaite des bagues conductrices annulaires 101, 102, 103 lorsque, au montage de la fiche 10, celles-ci sont placées sur les surfaces latérales. A cet effet, la courbure des surfaces latérales et la courbure intérieure des bagues doivent être identiques. La Figure 4 est une vue plus détaillée des surfaces latérales 213a, 213b.

Le raccordement électrique entre les fils 21, 22, 23 de liaison et les bagues conductrices annulaires 101, 102, 103 peut être réalisée de la manière suivante.

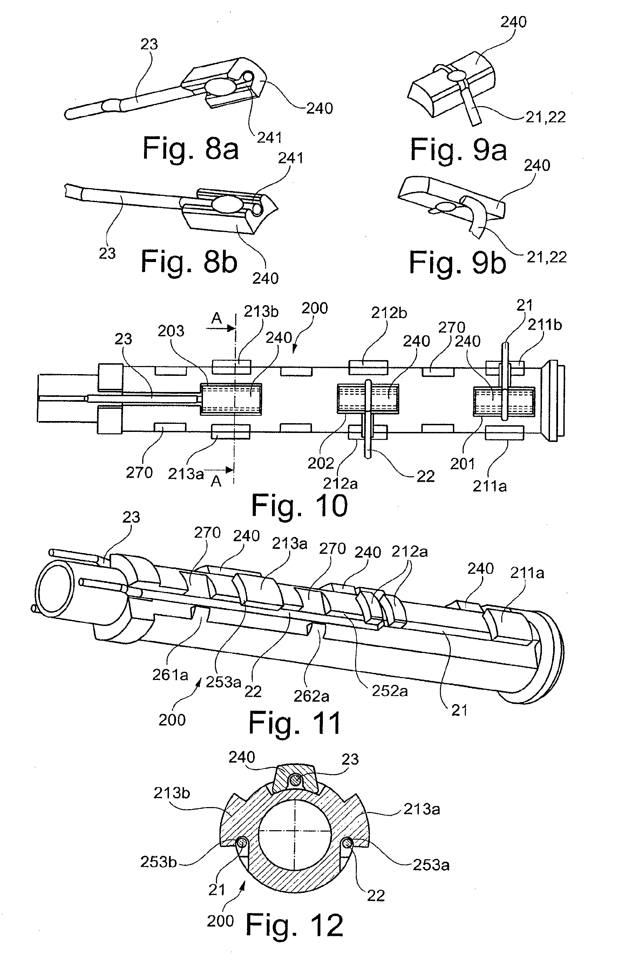

Dans un premier temps, conformément aux Figures 8a, 8b, 9a, 9b, les fils 21, 22, 23 de liaison sont reliés à des cosses conductrices intermédiaires, référencées génériquement 240, destinées à être disposées à l'intérieur de logements 201, 202, 203 prévus sur le noyau central 200 et que l'on peut voir plus en détail sur les Figures 3, 4, 6, 10.

Les cosses 240 sont réalisées dans un matériau conducteur biocompatible, par exemple de l'acier inox 316 SS ou MP35N. Les fils de liaison 21, 22, 23 sont de préférence protégés par une gaine protectrice isolante en éthylène tétrafluoroéthylène (ETFE) ou en polytétrafluoroéthylène (PTFE), les fils étant alors dénudés à leur extrémité reliée à la cosse intermédiaire 240.

Les cosses 240 peuvent être usinées.

Compte tenu de la position des logements 201, 202, 203 sur le noyau central 200, c'est-à-dire au niveau de l'emplacement des bagues annulaires 101, 102, 103, la courbure extérieure de la cosse 240 doit être compatible avec la courbure intérieure des bagues.

Deux versions de la cosse 240 sont proposées. La version des Figures 8a, 8b permet d'aligner directement le fil 23 de liaison dans l'axe du noyau 200. Dans ce cas, afin de permettre une introduction plus facile du fil 23 de liaison, la cosse intermédiaire 240 présente une rainure 241 d'insertion. Dans la version des Figures 9a, 9b, les fils 21, 22 sont placés transversalement, ce qui oblige à les courber et les plier pour les réaligner dans l'axe du noyau 200 et les rediriger vers le corps 20 de sonde.

La connexion électrique entre les fils 21, 22, 23 de liaison et la cosse intermédiaire 240 peut être réalisée par soudure laser, soudure électrique ou toute autre technologie apte à relier entre elles deux pièces métalliques.

Ensuite, les cosses 240 munies de leur fil de liaison respectif sont placées dans les logements 201, 202, 203. Les fils 21, 22 qui sortent transversalement à l'axe du noyau 200 sont introduits dans les fentes formées sur les surfaces latérales 211 b et 212a, puis courbés et pliés de manière à ce qu'ils s'étendent le long du noyau 200, parallèlement à son axe, comme le montre la Figure 11.

Cette opération d'assemblage peut être effectuée, sous binoculaire, à l'aide de l'outillage de maintien représenté à la Figure 5, où le noyau central 200 est placé en appui horizontal sur une platine évidée 2 par des ailes latérales 230a, 230b disposées en bout sur le noyau.

Selon une caractéristique avantageuse, le noyau 200 est muni de moyens permettant de maintenir les fils 21, 22 de liaison dans la position qu'ils occupent sur la Figure 11, à savoir contre le noyau et parallèles à son axe. Ces moyens de maintien comprennent, de manière générale, des encoches longitudinales ménagées sur les surfaces latérales de centrage. Par exemple, la vue en coupe de la Figure 12 montre des encoches longitudinales 253a, 253b ménagées dans les surfaces latérales 213a, 213b de centrage, ces encoches étant destinées à appliquer et maintenir respectivement les fils 21, 22 de liaison contre le noyau central 200. Comme l'indique la Figure 11, le fil 22 de liaison est également maintenu par l'encoche longitudinale 252a de la surface latérale 212a.

Afin de parfaire le maintien en position des fils de liaison, des deuxièmes surfaces latérales de centrage sont prévues sur le noyau central 200 pour retenir les fils 21, 22 de liaison dans les encoches longitudinales après qu'ils y aient été introduits. Sur la Figure 11 par exemple, les deuxièmes surfaces latérales de retenue du fil 22 sont référencées 261 a, 262a. D'autres surfaces latérales de ce type, non visibles sur les dessins, sont présentes symétriquement pour retenir le fil 21 dans les encoches correspondantes.

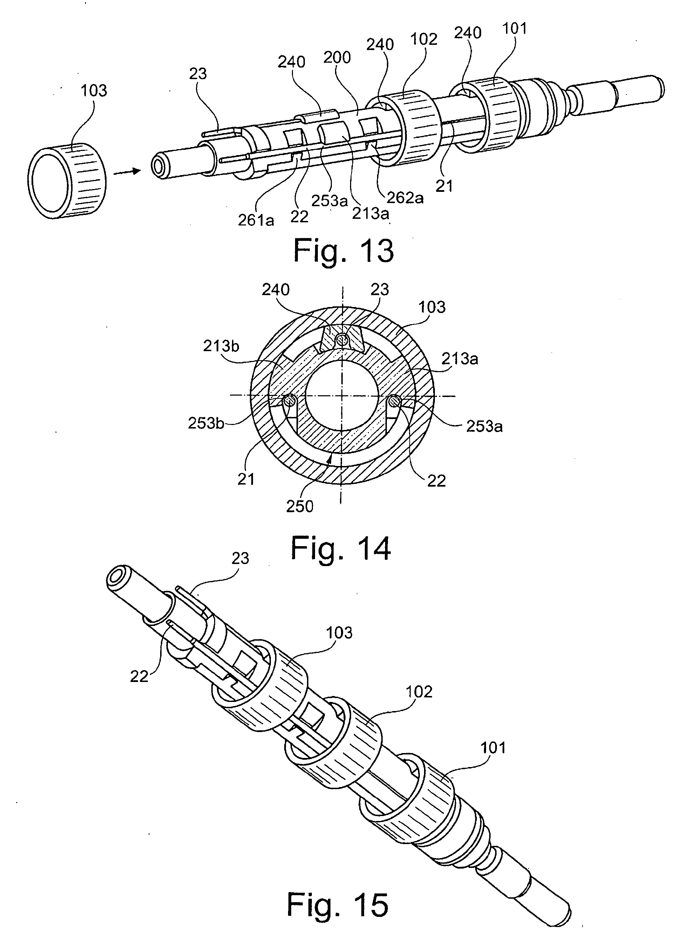

Conformément à la Figure 13, les bagues annulaires conductrices 101, 102, 103 sont alors mises en place en les faisant coulisser le long de l'axe du noyau 200 jusqu'à les amener en regard de la cosse 240 à laquelle elles doivent être électriquement reliées. La Figure 14 montre sur une vue en coupe la disposition finale de la bague conductrice 103 autour du noyau 200. Le noyau central 200 équipé de l'ensemble des bagues conductrices est représenté sur la Figure 15.

Les bagues 101, 102, 103 sont ensuite soudées aux cosses intermédiaires 240 par soudure laser, électrique ou toute autre technologie. Cette opération peut être réalisée bague après bague ou bien sur les trois bagues placées dans un premier temps dans un outillage de positionnement. La liaison par soudure ne doit pas affecter l'état de surface et la cylindricité des bagues. Pour cela, on peut, par exemple, réaliser une soudure sur le côté de la bague, en inclinant l'ensemble formé par le noyau 200, les fils conducteurs 21, 22, 23 reliés au cosses 240 et les bagues 101,102,103.

Cette étape constitue un procédé parfaitement maîtrisé et contrôlable, rendu simplifié par le noyau central 200 qui joue en partie le rôle d'outillage interne.

Les bagues conductrices ont un diamètre extérieur donné par la norme ISO 27 186. Leur positionnement est primordial, tout comme leur coaxialité. Comme on l'a déjà mentionné, la coaxialité est obtenue grâce aux surfaces latérales de centrage sur lesquelles les bagues viennent en appui. Quant au positionnement longitudinal, il peut être assuré par des épaulements 221 a, 221 b, 222a, 222b, 223a, 223b portés respectivement par les surfaces latérales 211 a, 211 b, 212a, 212b, 213a, 2123b que l'on peut voir sur la Figure 6, et contre lesquels les bagues 101, 102, 102 viennent en butée.

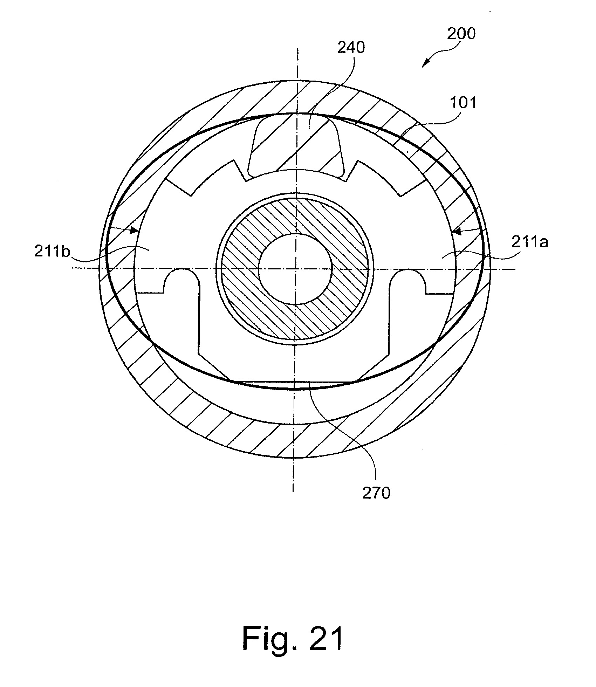

Dans ce cas, on prévoit que le noyau central 200 présente un méplat longitudinal 270, représenté sur la Figure 7, permettant une introduction des bagues conductrices sur les surfaces latérales de centrage par déformation élastique réversible. Comme le montre la Figure 21, une bague conductrice, telle que la bague 101 peut de cette manière être amenée en position en franchissant successivement les épaulements 223a, 223b, puis les épaulements 222a, 222b.

Enfin, la dernière étape consiste à remplir de matériau isolant les espaces 111, 112, 113, 114 de la Figure 2 existant entre les bagues 101, 102, 103 afin de réaliser les zones 31, 32, 33, 34 isolantes intercalaires séparant alternativement les zones 11, 12, 13 de contact montrées sur la Figure 1. Dans cette opération, on doit veiller à ce que la fiche 10 de connexion soit parfaitement isodiamètre en respectant la norme ISO 27 186, et qu'elle garantisse l'étanchéité du système, laquelle résulte, comme on l'a vu plus haut, de la qualité du contact de la fiche 10 avec des joints disposés dans la cavité du générateur prévue pour recevoir la fiche.

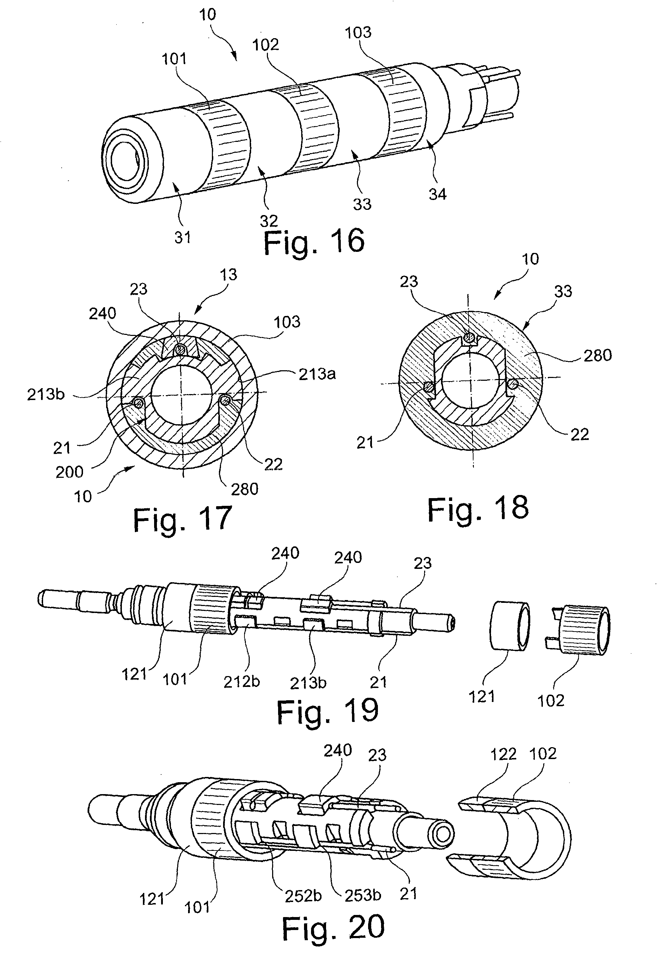

Le remplissage des espaces 111, 112, 113, 114 peut être réalisé, par exemple, par surmoulage ou injection de colle ou de matière plastique, ou de tout autre matériau isolant. Les Figures 17 et 18 montrent respectivement une coupe de la fiche 10 de connexion à travers la zone 13 de contact et une coupe à travers la zone isolante 33 après surmoulage avec un matériau isolant 280.

Selon une variante proposée sur la Figure 19, l'isodiamètre recherché pour la fiche 10 est obtenu par un empilage d'anneaux isolants, tels que ceux référencés 121, 122 sur la Figure précitée, et de bagues conductrices, la coaxialité de l'ensemble des anneaux et des bagues étant assurée par les surfaces latérales de centrage précédemment décrites, la liaison entre une bague conductrice et un sous-ensemble formé par un fil de liaison et la cosse correspondante étant réalisée avant chaque insertion d'un anneau isolant.

La seconde variante de la Figure 20 met en oeuvre des binômes constitués d'un anneau isolant surmoulé sur une bague conductrice, tels que les binômes anneau 121/bague 101 et anneau 122/bague 102, chaque binôme étant relié par la bague conductrice à un sous-ensemble fil de liaison/cosse avant l'introduction d'un nouveau binôme.

En conclusion, on comprend que l'invention permet non seulement de simplifier grandement l'assemblage d'une fiche de connexion de sonde multipôle, mais également d'avoir un contrôle visuel à chaque étape de l'assemblage. L'opérateur a donc une meilleure maîtrise à chaque étape du procédé.

De plus, la fiche de connexion selon l'invention peut à la fois être réalisée directement sur un corps de sonde existant ou être relié à la sonde a posteriori. Ceci permet au besoin de sous-traiter la fabrication de la fiche et de l'adapter à n'importe quel corps de sonde.

## Revendications

1. Une fiche (10) de connexion électrique pour une sonde multipôle (1) de dispositif médical implantable actif, cette fiche présentant une surface extérieure cylindrique comprenant une pluralité de zones (11, 12, 13) de contact électrique annulaires réparties axialement et formées de bagues cylindriques conductrices (101, 102, 103), les zones de contact électrique étant séparées alternativement par une pluralité de zones cylindriques isolantes intercalaires (31, 32, 33, 34),
cette fiche comprenant en outre un noyau central monobloc isolant (200) de forme générale cylindrique présentant une pluralité de surfaces latérales cylindriques de centrage coaxiales (211 a, 211 b, 212a, 212b, 213a, 213b), une bague conductrice étant placée sur au moins une surface latérale de centrage,
fiche **caractérisée en ce que** le noyau central comprend une pluralité de logements (201, 202, 203), **en ce qu'**elle comprend en outre une pluralité correspondante de cosses conductrices intermédiaires (240) reçues dans lesdits logements et sur lesquelles sont soudés des fils de liaison (21, 22, 23), et **en ce que** les cosses sont soudées à des bagues conductrices respectives.

2. La fiche de connexion de la revendication 1, dans laquelle les cosses intermédiaires présentent une rainure (241) d'insertion du fil de liaison, alignée dans l'axe du noyau ou transversalement à cet axe.

3. La fiche de connexion de la revendication 1, dans laquelle les surfaces latérales de centrage comportent un épaulement (221 a, 221 b, 222a, 222b, 223a, 223b) de positionnement longitudinal des bagues conductrices.

4. La fiche de connexion de la revendication 3, dans laquelle le noyau central présente un méplat longitudinal (270) pour une introduction des bagues conductrices sur les surfaces latérales de centrage par déformation élastique réversible.

5. La fiche de connexion de la revendication 1, dans laquelle les zones isolantes intercalaires (31, 32) sont constituées par des bagues isolantes (121, 122).

6. La fiche de connexion de la revendication 5, dans laquelle une bague isolante (121, 122) est surmoulée sur une bague conductrice (101, 102).

7. La fiche de connexion de la revendication 1, dans laquelle le noyau central est réalisé par moulage d'un matériau isolant.

8. La fiche de connexion de la revendication 1, comprenant des moyens (252a, 253a, 253b) de maintien de fils (21, 22) de liaison conducteurs le long du noyau central cylindrique, ménagés sur les surfaces latérales de centrage.

9. La fiche de connexion de la revendication 8, dans laquelle les moyens de maintien comprennent une encoche longitudinale (252a, 253a, 253b) ménagée dans les surfaces latérales (212a, 213a, 213b) de centrage et destinée à appliquer un fil de liaison contre le noyau cylindrique.

10. La fiche de connexion de la revendication 9, dans laquelle les moyens de maintien comprennent en outre au moins une deuxième surface latérale (261 a, 262a) de centrage destinée à retenir un fil de liaison dans au moins une encoche longitudinale (252a, 253a).

11. La fiche de connexion de la revendication 1, dans laquelle les zones isolantes intercalaires (31, 32, 33, 34) sont réalisées par surmoulage ou injection d'un matériau isolant (280).

## Patentansprüche

1. Elektrischer Anschlussstift (10) für eine multipolare Sonde (1) einer aktiven implantierbaren medizinischen Vorrichtung, wobei dieser Anschlussstift eine zylindrische Außenfläche aufweist, die mehrere ringförmige elektrische Kontaktbereiche (11, 12, 13) aufweist, die axial verteilt sind und von leitfähigen zylindrischen Ringen (101, 102, 103) gebildet werden, wobei die elektrischen Kontaktbereiche abwechselnd durch mehrere isolierende zylindrische Zwischenbereiche (31, 32, 33, 34) getrennt sind,
wobei dieser Anschlussstift außerdem einen isolierenden einstückigen Mittelkern (200) von allgemein zylindrischer Form aufweist, der mehrere koaxiale zylindrische seitliche Zentrierflächen (211a, 211b, 212a, 212b, 213a, 213b) aufweist, wobei ein leitfähiger Ring auf mindestens einer seitlichen Zentrierfläche angeordnet ist,
wobei der Anschlussstift **dadurch gekennzeichnet ist, dass** der Mittelkern eine Vielzahl von Aufnahmen (201, 202, 203) aufweist, dadurch, dass er außerdem eine entsprechende Vielzahl von leitfähigen Zwischen-Kabelschuhen (240) aufweist, die in den Aufnahmen aufgenommen sind und an denen Verbindungsdrähte (21, 22, 23) angeschweißt sind, und dadurch, dass die Kabelschuhe an jeweilige Ringe angeschweißt sind.

2. Anschlussstift nach Anspruch 1, wobei die Zwischen-Kabelschuhe eine Nut (241) zum Einsetzen des Verbindungsdrahtes aufweisen, die entlang der Achse des Kerns oder quer zu dieser Achse ausgerichtet ist.

3. Anschlussstift nach Anspruch 1, wobei die seitlichen Zentrierflächen einen Ansatz (221a, 221b, 222a, 222b, 223a, 223b) zur Positionierung der leitfähigen Ringe in Längsrichtung aufweisen.

4. Anschlussstift nach Anspruch 3, wobei der Mittelkern eine Längsabflachung (270) für eine Anbringung der leitfähigen Ringe auf den seitlichen Zentrierflächen durch reversible elastische Verformung aufweist.

5. Anschlussstift nach Anspruch 1, wobei die isolierenden Zwischenbereiche (31, 32) aus isolierenden Ringen (121, 122) bestehen.

6. Anschlussstift nach Anspruch 5, wobei ein isolierender Ring (121, 122) auf einen leitfähigen Ring (101, 102) aufgeformt ist.

7. Anschlussstift nach Anspruch 1, wobei der Mittelkern durch Formen eines isolierenden Materials hergestellt ist.

8. Anschlussstift nach Anspruch 1, welcher Mittel (252a, 253a, 253b) zum Halten leitender Verbindungsdrähte (21, 22) entlang des zylindrischen Mittelkerns aufweist, die an den seitlichen Zentrierflächen ausgebildet sind.

9. Anschlussstift nach Anspruch 8, wobei die Mittel zum Halten eine Längsvertiefung (252a, 253a, 253b) aufweisen, die in den seitlichen Zentrierflächen (212a, 213a, 213b) ausgebildet ist und dazu bestimmt ist, einen Verbindungsdraht an den zylindrischen Kern anzudrücken.

10. Anschlussstift nach Anspruch 9, wobei die Mittel zum Halten außerdem mindestens eine zweite seitliche Zentrierfläche (261a, 262a) aufweisen, die dazu bestimmt ist, einen Verbindungsdraht in mindestens einer Längsvertiefung (252a, 253a) festzuhalten.

11. Anschlussstift nach Anspruch 1, wobei die isolierenden Zwischenbereiche (31, 32, 33, 34) durch Aufformung oder Einspritzung eines isolierenden Materials (280) hergestellt sind.

## Claims

1. An electric connection plug (10) for a multi-pole lead (1) of an active implantable medical device, this plug having a cylindrical external surface comprising a plurality of annular electric-contact areas (11, 12, 13), distributed axially and formed of conductive cylindrical rings (101, 102, 103), the electric-contact areas being alternately separated by a plurality of separating insulating cylindrical areas (31, 32, 33, 34),
this plug further comprising a generally-cylindrical insulating one-piece central core (200) having a plurality of coaxial cylindrical lateral centering surfaces (211a, 211b, 212a, 212b, 213a, 213b), a conductive ring being placed on at least one lateral centering surface,
the plug being **characterized in that** the central core comprises a plurality of accommodations (201, 202, 203), **in that** it further comprises a corresponding plurality of intermediate conductive terminals (240) received in said accommodations and on which are welded connecting wires (21, 22, 23), and **in that** the terminals are welded to respective conductive rings.

2. The connection plug of claim 1, wherein the intermediate terminals have a groove (241) for insertion of the connecting wire, aligned in the axis of the core or transverse to this axis.

3. The connection plug of claim 1, wherein the lateral centering surfaces include a shoulder (221a, 221b, 222a, 222b, 223a, 223b) for longitudinal positioning of the conductive rings.

4. The connection plug of claim 3, wherein the central core has a longitudinal flat (270) for introduction of the conductive rings on the lateral centering surfaces through reversible elastic deformation.

5. The connection plug of claim 1, wherein the separating insulating areas (31, 32) are consisted by insulating rings (121, 122).

6. The connection plug of claim 5, wherein an insulating ring (121, 122) is moulded over a conductive ring (101, 102).

7. The connection plug of claim 1, wherein the central core is made by moulding of an insulating material.

8. The connection plug of claim 1, comprising means (252a, 253a, 253b) for holding conductive connecting wires (21, 22) along the cylindrical central core, arranged on the lateral centering surfaces.

9. The connection plug of claim 8, wherein the holding means comprise a longitudinal notch (252a, 253a, 253b) arranged in the lateral centering surfaces (212a, 213a, 213b) and intended to apply a connecting wire against the cylindrical core.

10. The connection plug of claim 9, wherein the holding means further comprise at least one second lateral centering surfaces (261a, 262a) intended to hold a connecting wire in at least one longitudinal notch (252a, 253a).

11. The connection plug of claim 1, wherein the separating insulating areas (31, 32, 33, 34) are made by over-moulding or injection of an insulating material (280).
